# EUROPEAN PATENT APPLICATION

(11) **EP 3 695 776 A1**
(43) Date of publication of application: **19.08.2020**
(21) Application number: 19156851.8
(22) Date of filing: 13.02.2019
(51) Int. Cl.: A61B 5/00, A61B 5/08, G08B 21/02, G16H 50/30, G16H 50/20

(54) **A METHOD AND SYSTEM FOR GENERATING A RESPIRATION INSTABILITY SIGNAL**

(71) Applicant: Koninklijke Philips N.V., 5656 AG Eindhoven (NL)
(72) Inventor: JOSHI, Rohan, 5656 AE Eindhoven (NL)
(74) Representative: de Haan, Poul Erik

(57) **Abstract**

A method and processing system adapted to monitor respiratory instability by directly calculating a measure of periodicity and amplitude regularity, which is effectively a measure of disorganization, of a respiratory waveform. Normal respiration is a somewhat periodic signal whereas complete cessation of breathing would result in the respiratory signal reflecting measurement noise (i.e. aperiodic with minimal amplitude regularity). Thus, the measure is responsive to changes in the subject's breathing, and can distinguish between normal and abnormal breathing patterns.

## Description

### FIELD OF THE INVENTION

The present invention relates to the field of subject monitoring, and in particular to the monitoring of respiration of a subject.

### BACKGROUND OF THE INVENTION

In a clinical environment, such as a hospital, the respiration of a subject or patient is commonly monitored, as respiration is an important indicator of the condition of the subject.

Various techniques for monitoring a respiration rate have been proposed, such as using pressure sensors, chest impedance sensors (e.g. using electrocardiogram, ECG, electrodes), plethysmography sensors, electromyography (EMG) sensors and so on to obtain a signal responsive to respiration of the subject. ECG electrodes for detecting chest impedance are the most commonly used in current clinical settings. Other techniques for monitoring respiration instead monitor parameters affected by respiration, such as oxygen saturation level (SpO₂).

There is an ongoing desire to detect, measure or monitor for respiration difficulty or problems in subjects, and to generate an alert for a clinician in response to respiration difficulty/problems. For example, apnea or cessation of breathing is a particular concern, especially in the neonatal clinical environment.

However, typical methods of identifying respiration difficulty or problems usually detect low or high respiratory rates, the absence of breathing for a certain duration of time or the drop of a subject's oxygen saturation below a certain threshold. These approaches are restricted in that they can fail to recognize when a subject's breathing is merely inefficient (e.g. due to obstructive or mixed apneas) for a period of time or if respiratory cessations are only short in length, or interspersed by gasps or short breaths. Thus, existing methods of identifying respiration difficulty suffer from lack of sensitivity and accuracy.

Not only this, but conventional sensors for directly measuring respiration rate suffer from a lack of reliability and sensitivity, due at least to cardiac artifacts and subject motion. In particular, the beating of a heart or movement of the body may resemble a breath to some sensors, such as chest impedance measures. Thus, attempts to detect respiration difficulty/problems may fail due to an inaccurate respiratory sensor.

There is therefore a desire to improve methods of detecting respiratory difficulties and/or problems, or to produce signals that accurately respond to difficulties in a subject's breathing.

### SUMMARY OF THE INVENTION

The invention is defined by the claims.

According to examples in accordance with an aspect of the invention, there is provided a method of generating a respiratory instability signal representative of a monitored subject's respiratory instability.

The method comprises: receiving a subject monitoring signal responsive to respiration of the subject; and processing the subject monitoring signal using a function that derives a measure of periodicity and amplitude regularity during respiration, to thereby generate a respiratory instability signal representative of a monitored subject's respiratory instability.

The present invention proposes to monitor respiratory instability, representing potential breathing difficulties or problems, by directly calculating a measure of periodicity and amplitude regularity of a signal responsive to respiration of the subject. For example, a respiratory waveform may be processed using a particular function to generate a signal indicative of disorganization (i.e. changes in the periodicity and regularity) or a measure of randomness in the subject's respiration.

Use of such a function allows for the presence or absence of irregular breathing patterns to be quantified. The respiratory instability signal thereby provides an indicator of the subject breathing difficulty and identifies changes in their breathing. In particular, a low measure (obtained by the function) may indicate that the subject's respiration is periodic and predictable, i.e. subject has no respiration difficulty, whereas a high measure (obtained by the function) may indicate that the subject's respiration is disorganized or absent, i.e. the subject has respiration difficulty.

The present invention recognizes that the respiratory instability signal can provide an early marker of respiration difficulty, and responds to changes in a subject's respiration more promptly and/or reliably than existing methods of monitoring a subject's respiration. Thus, an improved measure or indicator of the subject's respiration difficulties is generated.

Moreover, the respiratory instability signal will be able to detect short cessations/interruptions in a subjects breathing as well as inefficient breathing techniques, as these will induce changes in the periodicity or amplitude regularity of the respiration.

Preferably, the function that derives a measure of periodicity and amplitude regularity during respiration is an entropy function.

In the context of the present invention, an entropy function is any function that processes time-series input data to provide one or more output values quantifying the regulatory and/or the unpredictability of fluctuations of the time-series input data. In other words, an entropy function takes into account both signal periodicity and signal amplitude to determine its output. The entropy function may therefore provide a measure of disorganization of the time-series input data. Examples of suitable entropy functions include an approximate entropy (ApEn), sample entropy (SampEn), distribution entropy (DistEn) and so on. Other entropy functions will be apparent to the skilled person.

The method may comprise a step of filtering the subject monitoring signal using a bandpass filter before processing the subject monitoring signal using the function.

A bandpass filter may be used to minimize the influence of other regular bodily functions (e.g. heartbeat or swallowing) on the subject monitoring signal. Thus, respiratory information may be extracted or isolated from the subject monitoring signal. This improves an accuracy of the respiratory instability signal.

The precise parameters of the bandpass filter may depend upon the environment in which the method is implemented. For example, in a neonatal environment, the bandpass filter may isolate frequencies within a range having a lower bound of 0.1 and/or an upper bound of 2Hz, such as a range of from 0.45 to 1.45Hz. In an adult-care environment, the bandpass filter may isolate frequencies within a range having a lower bound of 0.13 and/or an upper bound of 0.35Hz, such as a range of from 0.15 to 0.30Hz. This takes account of differences in expected breathing rates between neonatal and adult subjects. Generally speaking, the bandpass filter may isolate frequencies within the range of 0.1Hz to 2Hz.

The step of processing the subject monitoring signal may comprise iteratively: obtaining a window of the subject monitoring signal, a window being a windowed portion of the subject monitoring signal captured over a first predetermined length of time; and processing the window using the function to thereby generate a respiratory instability value for the respiratory instability signal.

Iteratively obtaining a value for the respiration instability signal means that values for the respiration instability signal can be continuously generated, and enables the respiration instability signal to be generated in "real-time". Using a window of the subject monitoring signal to generate successive values of the respiratory instability signal provides an effective and resource-efficient method of determining a value for the respiration instability signal, e.g. as pipeline techniques may be used.

Preferably, a start time of a window of the subject monitoring signal obtained in any given iteration is after a start time of a window of the subject monitoring signal obtained in an immediately previous iteration. Each window spans from a respective start time to a respective end time, as would be well understood by the skilled person.

Thus, windows used in different iterations may overlap one another, so that the start time of a window in any given iteration may be within the window of the subject monitoring signal obtained in an immediately previous iteration. Thus, the values of the respiratory instability signal may be an output of a function iteratively performed on a moving window of the subject monitoring signal.

In at least one embodiment, the step of obtaining a window of the subject monitoring signal comprises obtaining a most recent portion, of the first predetermined length of time, of the subject monitoring signal.

Thus, the window may represent the most recently available data for processing with a function. This means that the respiratory instability signal can represent the most recent parameters of the subject, and therefore improve the speed at which respiration instability/problems is/are identified. This reduces a likelihood that the patient will deteriorate due to respiration difficulty.

In some embodiments, the first predetermined length of time is no less than 10 seconds. Preferably, the first predetermined length of time is no less than 13 seconds, for example, no less than 15 seconds.

According to examples in accordance with an aspect of the invention, there is provided a method of selectively generating a respiratory instability alert. The method comprises: generating a respiratory instability signal using any previously described method; monitoring the respiratory instability signal to detect when a parameter of the magnitude of the respiratory instability signal goes above a predetermined threshold; and in response the parameter of the magnitude going above the predetermined threshold: determining a threshold breach period, being a measure of how long the parameter of the magnitude remains above the predetermined threshold; and generating a respiratory instability alert if the threshold breach period is greater than a predetermined time period.

In this embodiment, a respiratory instability alert is generated when the respiratory instability signal remains above a predetermined threshold for a certain period of time. Such a scenario indicates that the subject may be having respiratory difficulties (e.g. apnea or reduced airflow). By generating an alert, a likelihood that respiratory difficulty of the subject will be overlooked is reduced, thereby improving subject outcome.

Preferably, the respiratory instability alert comprises or initiates a clinician perceptible alert, such as an audio, visual and/or haptic alarm. This means that clinician can be alerted when there is respiratory difficulty detected in the subject, reducing the likelihood that the subject will enter a respiratory difficulty without this being recognized.

In some embodiments, the predetermined time period is no less than 10 seconds. In some preferred embodiments, the parameter of the magnitude is a value of the magnitude of the respiratory instability signal.

In another embodiments, the parameter may comprise a moving average (e.g. being an average captured over a certain time period, such as 1 second, 2 seconds and so on). Such embodiments further reduce the effect of noise or inaccurate monitoring on the alert, reducing the likelihood that a false alert will be generated.

According to examples in accordance with an aspect of the invention, there is provided another method of selectively generating a respiratory-affecting disease alert. The method comprising: generating a respiratory instability signal using any appropriate described method; obtaining a window of the respiratory instability signal, the window of the respiratory instability signal being a windowed portion of the respiratory instability signal over a second predetermined length of time; processing the window of the respiratory instability signal to determine whether or not to generate the respiratory-affecting disease alert based on whether the window of the respiratory instability signal meets a predetermined criterion; and generating a respiratory-affecting disease alert based on an outcome of the processing the window of the respiratory instability signal.

One early indicator of some diseases, such as sepsis, is a dampened respiratory drive. It is recognized that the stability of the respiratory drive can be derived from the respiratory instability signal obtained using a function that derives a measure of periodicity and amplitude regularity during respiration. In particular, windowing (or optionally segmenting) a respiratory instability signal, and processing each window separately allows a long-term analysis of the respiratory instability signal to take place.

Preferably, the respiratory-affecting disease alert comprises or initiates a clinician perceptible alert, such as an audio, visual and/or haptic alarm. This means that clinician can be alerted when it is predicted that there is a disease causing respiratory difficulty in the subject, reducing the time over which the subject progresses with the disease before being identified.

The step of processing the window may comprises: taking an average of the magnitude of the window of the respiratory instability signal; and determining to generate a respiratory-affecting disease alert if the average of the magnitude of the respiratory instability signal is greater than a predetermined average magnitude threshold.

The mean or average of the respiratory instability signal has been identified as being a highly accurate indicator of a likelihood that a respiratory-affecting disease will occur.

In an alternative embodiment, processing the window may comprise determining a (relative) length of time that the magnitude of the window of the respiratory instability signal is above a predetermined value and determining to generate a respiratory-affecting disease alert if the length of time is above a predetermined disease-indicating length of time. The predetermined disease indicating length of time may be expressed as a proportion of the length of the window of the respiratory instability signal (e.g. no less than 90% or no less than 95%).

Preferably, the second predetermined length of time is no less than 1 hour. The inventors have recognized, in particular, that features of the respiration instability signal over a longer period of time (>1 hour) are particularly representative and responsive to the development of a respiratory-affecting disease, which typically takes place over a period of time (i.e. over at least an hour). Preferably, the second predetermined time period is no less than 2 hours, for example, no less than 3 hours.

According to examples in accordance with an aspect of the invention, there is provided a computer program comprising code means for implementing any described method when said program is run on a computer.

According to examples in accordance with an aspect of the invention, there is also provided a processing system for generating a respiratory instability signal representative of a monitored subject's respiratory instability. The processing system being adapted to: receive a subject monitoring signal responsive to respiration of the subject; and process the subject monitoring signal using a function that derives a measure of periodicity and amplitude regularity during respiration, to thereby generate a respiratory instability signal representative of a monitored subject's respiratory instability.

The processing system may be integrated into a subject monitoring system. Thus, there may be subject monitoring system for generating a respiratory instability signal, the patient monitoring system comprising: one or more subject monitoring sensors adapted to generate a subject monitoring signal responsive to respiration of the subject; and the processing system herein described adapted to receive the subject monitoring system generating by the one or more subject monitoring sensors.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a flowchart illustrating a method according to an embodiment;
Figure 2 illustrates waveforms for understanding an underlying concept of embodiments;
Figure 3 is a flow chart illustrating a method according to another embodiment;
Figure 4 is a flow chart illustrating a method according to yet another embodiment;
Figure 5 is a graph illustrating a use-case scenario for an embodiment; and
Figure 6 illustrates a subject monitoring system according to an embodiment;

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention proposes to monitor respiratory instability by directly calculating a measure of periodicity and amplitude regularity, which is effectively a measure of disorganization, of a respiratory waveform. Normal respiration is a somewhat periodic signal whereas complete cessation of breathing would result in the respiratory signal reflecting measurement noise (i.e. aperiodic with minimal amplitude regularity). Thus, the measure is responsive to changes in the subject's breathing, and can distinguish between normal and abnormal breathing patterns.

Thus, embodiments are based on the realization that "normal" or clinically acceptable breathing is typically periodic and of a regular amplitude (i.e. each period has approximately the same amplitude), whereas "abnormal" or clinically unacceptable breathing is either irregular or absent, where absent breathing leads to an aperiodic and irregular amplitude signal due to noise. Thus, a measure of periodicity and amplitude regularity, such as an entropy measure, can distinguish between normal or abnormal breathing.

Embodiments may be employed in a respiratory monitor to more quickly identify potential respiratory degradation. Embodiments may also be employed to detect long-term respiratory stability decline, which may be caused by the onset of a diseases that dampen the respiratory drive, such as sepsis. Embodiments may therefore be used in a clinical setting, such as a neonatal unit or a long-term intensive care unit or ward.

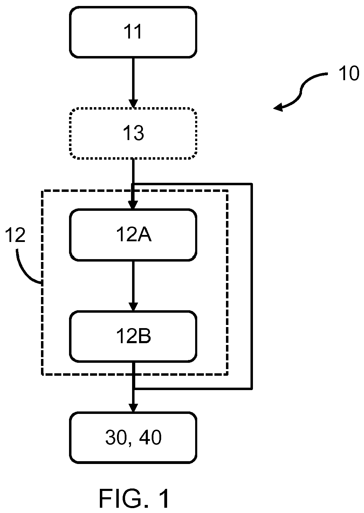

Figure 1 illustrates a method 10 according to an embodiment of the invention.

The method 10 comprises a step 11 of obtaining or receiving a subject monitoring signal responsive to respiration of the subject. The subject monitoring signal is any signal responsive to an inhalation and/or exhalation of a subject, such as a chest impedance measure, pressure sensor signal, measure of airflow, a measure from a plethysmography sensor, or a measure from electromyography (EMG) sensors.

The method 10 also comprises a step 12 of processing the subject monitoring signal using a function that derives a measure of periodicity and amplitude regularity during respiration. Processing the subject monitoring signal in this way generates a respiratory instability signal that is representative of a monitored subject's respiratory instability.

One suitable function for processing the subject monitoring signal is an entropy function. An entropy function outputs a measure that can be treated as a measure of the randomness or complexity of a signal. It has been recognized that such a measure is reflective of the respiratory instability of the subject. Examples of entropy functions include: Approximate Entropy (ApEn), sample entropy (SampEn), distribution entropy (DistEn) etc.

Step 12 may comprise, for example, a sub-step 12A obtaining a window of the subject monitoring signal, being an extract of the subject monitoring signal that spans a first predetermined period of time. Step 12 may further comprise a sub-step 12B processing the window of the subject monitoring signal using the function that derives a measure of periodicity and amplitude regularity. In particular, sub-step 12B may comprise processing the window to generate one or more values, and preferably a single value, indicative of a complexity of the window of the subject monitoring signal. For example, sub-step 12B may comprise processing the window of the subject monitoring signal using an entropy function.

The precise process used in step 12B will depend upon implementation details. By way of example, the window of the subject monitoring signal may be formed as an N-length series/vector of samples or data points. This series or vector may then be processed using an entropy function, such as Approximate Entropy (ApEn), to generate a single value for the respiratory instability signal. Other suitable entropy functions will be known to the skilled person, such as sample entropy (SampEn), distribution entropy (DistEn) and so on.

The necessary input parameters for an entropy function would be well known to the skilled person, and they would be readily capable of processing (a portion of) a subject monitoring signal to provide the necessary input parameters for the entropy function. Typically, the output of an entropy function is a numerical value ranging from 0 to 1 or from -1 to 1.

Step 12 may be iteratively repeated for different windows of the subject monitoring signal, to thereby generate different measures for the respiratory instability signal. Each window used in particular iteration preferably begins temporally after a window of a previous iteration begins. Thus, a start time for a subsequent window is after a start time for a previous window. This effectively means that a moving window of the subject monitoring signal is obtained and processed in iterations. In some embodiments, a window of a subsequent iteration may overlap a window of a previous iteration.

The length of the window is preferably greater than 10 seconds, for example, no less than 15 seconds. Thus, the first predetermined time period may be no less than 10s, for example, no less than 15 seconds.

The respiratory instability signal may be continually generated, i.e. in real-time. Thus, the step 12A of obtaining a window of the subject monitoring signal may comprise obtaining a most recent portion of the subject monitoring signal. Thus, an end time of the obtained window in step 12A may end at a current point in time (i.e. the most recently available data of the subject monitoring signal). This means that the respiratory instability signal reflects available data, improving the speed at which respiratory instability or respiratory failure events are identifiable.

In this way, the respiratory instability signal can be built up over time. In particular, variations in the respiratory instability signal indicates variations in the respiratory stability of the subject, and can thereby indicate respiratory failure events.

The respiratory instability signal can subsequently be processed, in a process 30, 40 to determine whether or not to generate a respiratory instability alert. Embodiments of such a process 30, 40 will be made clear later in the description.

In some embodiments, the method 10 may further comprise a step 13 of (preferably bandpass) filtering the subject monitoring signal prior to the processing step 12. This reduces the effect of noise on the respiratory instability signal, thereby increasing an accuracy of the respiratory instability signal. The filtering is designed to isolate the respiratory-relevant information from the subject monitoring signal. The characteristics of the filter may depend upon implementation details.

Preferably, the filter has an upper bound of no more than 2Hz, for example, no more than 1.5Hz. In some embodiment the filter may, additionally or alternatively, have a lower bound of no less than 0.10Hz. Thus, step 13 may comprise filtering the subject monitoring signal to isolate frequencies in the range of from 0.10Hz to 2Hz.

For improved performance in a neonatal environment, step 13 may comprise filtering the subject monitoring signal to isolate frequencies in the range of from 0.45Hz to 1.45Hz. As adults typically breathe at a slow rate, for improved performance in an adult clinical setting, step 13 may comprise filtering the subject monitoring frequency to isolate frequencies in the range of from 0.13Hz to 0.35Hz, e.g. from 0.15Hz to 0.30Hz. Other suitable embodiments will be apparent to the skilled person.

Step 13 may be omitted in some embodiments of the invention, for example, where the subject monitoring signal is only responsive to a respiration of the subject, such as from a respiratory monitor that directly measures airflow.

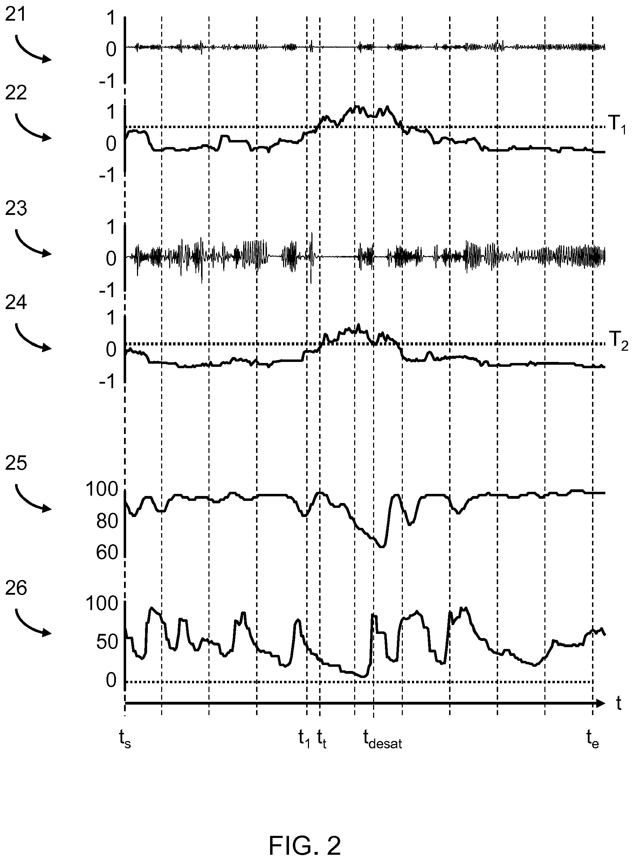

Figure 2 illustrates waveforms demonstrating the generation of the respiratory instability signal. Each waveform represents signals captured or otherwise representing a same period of time, beginning at a capture start time tₛ and ending at a capture end time tₑ. In this illustration, the time span between the capture start and capture end times is five minutes, which has been divided into ten 30-second segments for clarity.

A first waveform 21 illustrates a first bandpass filtered subject-monitoring signal, e.g. obtained from a chest impedance measurement. A second waveform 22 illustrates a first respiratory instability signal generated from the first waveform 21, using the method 10 previously described.

A third waveform 23 illustrates a second, different bandpass filtered subject-monitoring signal, e.g. obtained from a pressure sensor (such as a ballistography signal, BSG). A fourth waveform 24 illustrates a second respiratory instability signal generated from the third waveform 23, using the method 10 previously described.

For the sake of comparison to conventional methods of monitoring respiration, a fifth waveform 25 illustrates an oxygen saturation SpO₂ and a sixth waveform 26 illustrates a measured respiration rate. The measured respiration rate (respiratory rate 26) is obtained from one or both of the first and second subject-monitoring signals.

At a time t_{desat}, a desaturation event occurs (where an SpO₂ measurement dips below a clinically acceptable value, e.g. less than 80%). This indicates that it is likely a respiration interruption or problem has occurred, i.e. a respiratory failure event has occurred. However, the measured respiration rate 26 does not indicate that an apnea has occurred (as the respiration rate remains above zero throughout, as the cessation of breathing is relatively short). Thus, the monitoring respiration rate 26 alone may not be sufficient to detect or predict the onset of a respiratory failure event, and monitoring the SpO₂ is unable to predict the occurrence of the respiratory event - as it indicates when the event occurs.

However, the first 22 and second 24 respiratory instability signals both rise before the onset of the respiratory failure event, beginning to rise at a time t₁ - at which time the measure of the respiratory rate is within normal bounds. At a trigger time tt, both the first 22 and second 24 respiratory instability signals rise above a respective threshold value T₁, T₂, which can indicate that a respiratory failure event is predicted. The skilled person would be readily capable of setting appropriate threshold values for the respiratory instability signals.

In other words, the respiratory instability signal can provide an early indicator of the onset of a respiratory failure event, e.g. more than 30 seconds before the event occurs. Thus, the respiratory instability signal provides a good indicator of the respiratory instability of the subject, and thereby a good indicator of the probability that a respiratory failure event will occur.

Thus, the respiratory instability signal is an early marker of desaturation that can be used to create preemptive alarms and address alarm fatigue. Moreover, as expected, the respiratory instability signal remains low when the respiratory waveform is regular (i.e. respiration is "normal" or within clinically-acceptable bounds).

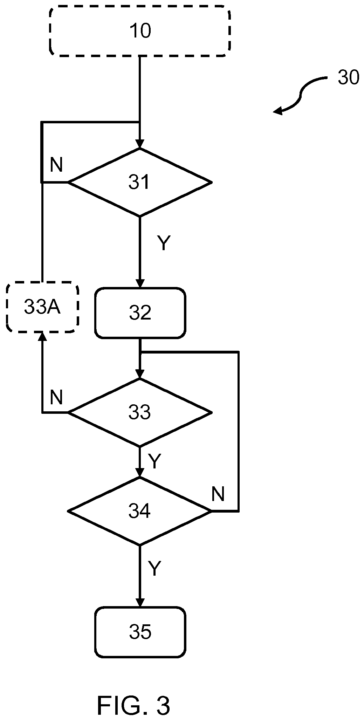

Figure 3 illustrates a method 30 of selectively generating a respiratory instability alert based on a respiratory instability signal generated according to a previously described method. The method 30 effectively monitors the respiratory instability signal to determine whether to generate an alert.

Method 30 comprises a step 31 of monitoring the respiratory instability signal to determine whether the respiratory instability signal is above a predetermined threshold. Thus, step 31 may comprise determining whether or not the magnitude of the respiratory instability signal is above the predetermined threshold.

In response to the magnitude of the respiratory signal not being above the predetermined threshold, the method repeats step 31.

In response to the magnitude of the respiratory instability signal going above the predetermined threshold (as determined in step 31), the method moves to a step 32 of starting a timer, which is intended to time how long the magnitude of the respiratory instability signal is above the predetermined threshold.

After performing step 32, a step 33 of re-determining whether the respiratory instability signal is still above the predetermined threshold is performed. In response to a positive determination (i.e. the magnitude is still above the predetermined threshold), the method moves to a step 34. Otherwise, the method reverts back to step 31. Optionally, when reverting back to step 31, the timer begun in step 32 may be stopped and optionally reset in a step 33A. Otherwise, one or more of the stopping and resetting steps may be performed in step 31 (before starting the timer).

Thus, steps 32 and 33 effectively determine a threshold breach period, being a measure of how long the parameter of the magnitude remains above the predetermined threshold. The threshold breach period is the length of time measured by the timer.

Step 34 comprises determining if the threshold breach period is greater than a predetermined time period. In response to the threshold breach period (i.e. the time on the timer) being greater than the predetermined time period, step 35 of generating an instability alert is performed. Otherwise, the method reverts back to step 33.

Thus, steps 34 and 35 effectively comprise a single step of generating a respiratory instability alert if the threshold breach period is greater than or equal to a predetermined time period. Otherwise, no respiratory instability alert is generated (i.e. if the threshold breach period is less the predetermined time period).

The length of the predetermined time period may vary depending upon implementation details (i.e. to strike a balance between reliability and sensitivity). However, for the sake of suitable reliability, the predetermined time period is preferably no less than 10 seconds, for example, no less than 15 seconds.

There may be an inverse relationship between the length of the predetermined time period and the length of the window used to obtain the respiratory instability signal. Thus, as the length of the window increases, so the length of the predetermined time period may decrease.

In this way, method 30 generates a respiratory instability alert if the magnitude of the respiratory instability signal remains above a predetermined threshold for at least a predetermined time period. This requirement for remaining above a predetermined time period reduces the likelihood of noise accidentally triggering a respiratory instability alert (e.g. which may occur if a respiratory instability alert is generated based only on an instantaneous magnitude), thereby improving a reliability of the respiratory instability alert.

The magnitude of the respiratory signal used in method 30 is preferably the instantaneous magnitude of the respiratory signal (i.e. the most recently available value of the respiratory instability signal).

However, the method 30 may be adapted to use other parameters of the magnitude of the respiratory signal, instead of the magnitude of the respiratory signal, such an average (over an immediately previous certain period of time, such as 1 second or 2 seconds) of the magnitude of the respiratory signal, a gradient of the magnitude of the respiratory signal, an average (over an immediately previous certain period of time, such as 1 second or 2 seconds) of the gradient of the magnitude of the respiratory signal and so on. The certain period of time may, for example, be no less than 1 second, for examples, no less than 2 seconds. In particular, the certain period of time may be equal to any disclosed "predetermined time period" above.

Thus, step 31 may alternatively comprise detecting when a parameter of the magnitude of the respiratory instability signal goes above a predetermined threshold, where the parameter may be: an average magnitude, instantaneous magnitude, average gradient, instantaneous gradient and so on.

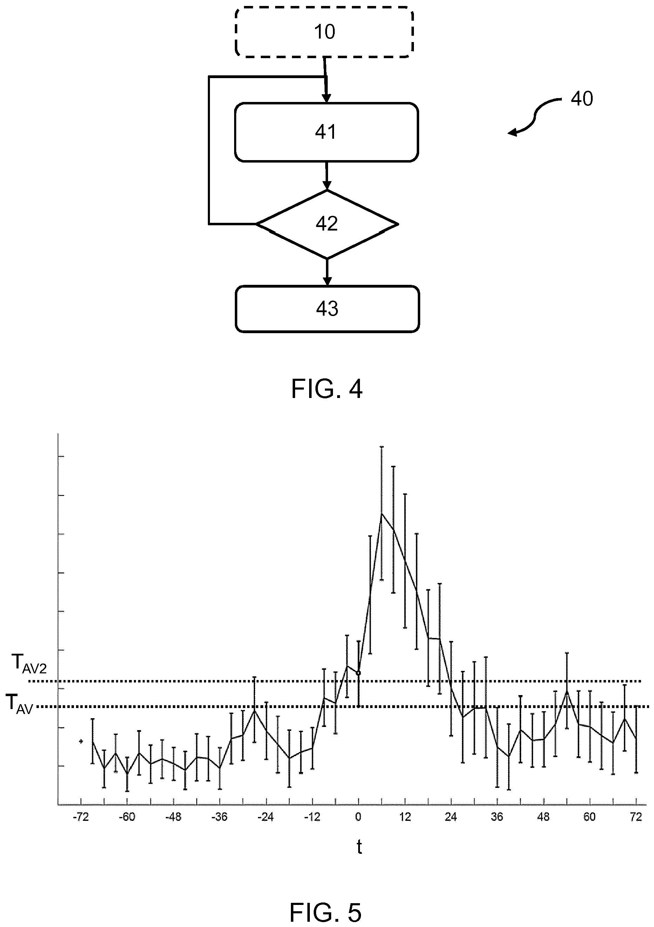

Figure 4 illustrates an alternative method 40 of monitoring the respiratory instability signal to determine whether or not to generate a respiratory instability alert.

The method 40 comprises a step 41 of obtaining a window of the respiratory instability signal. The window of the respiratory instability signal is a windowed portion of the respiratory instability signal over a second predetermined length of time.

The method 40 further comprises a step 42 of determining whether or not the window of the respiratory instability signal meets a predetermined criterion. Thus, step 42 comprises processing the window to decide whether one or characteristics of the respiratory signal (within that window) meet a predetermined criterion.

In response to step 42 determining that the window meets the predetermined criterion, a step 43 of generating a respiratory-affecting disease alert is performed. Otherwise, step 41 is repeated (i.e. to reobtain a new window of the respiratory instability signal).

The criterion used in step 42 may differ in different embodiments.

In one embodiment, the criterion of step 42 may be that the average magnitude of the respiratory instability signal within the window is above a predetermined average magnitude threshold.

Thus, in an embodiment, step 42 may comprise taking an average of the magnitude of the window of the respiratory instability signal and determining that the window of the respiratory signal meets the predetermined criterion if the average of the magnitude of the respiratory instability signal is greater than a predetermined average magnitude threshold. In other words, a respiratory-affecting disease alert may be generated if the average of a magnitude of the window of the respiratory instability signal is greater than a predetermined average magnitude threshold.

In another example, the criterion of step 42 may be that the total amount of time that a magnitude of the respiratory instability signal within the window is above a predetermined threshold for more than predetermined total amount of time. By way of example, the criterion of step 42 may be that the magnitude of the respiratory instability signal is above a predetermined threshold for more than a certain percentage length of the window (e.g. more than 50% or more than 75%, preferably more than 90% or 95%). The predetermined threshold may be no less than 50% of the maximum possible value of the respiratory instability signal (e.g. no less than 0.5 where the maximum value of the respiratory instability signal is 1.)

Numerous other criteria could be used in step 42. The underlying principle is that step 42 should determine whether the window of the respiratory instability signal deviates from a normal or conventional operation (e.g. where the measured instability would remain below a threshold).

The length of the second predetermined length of time is preferably no less than 1 hour, for example, no less than 3 hours, for example, no less than 6 hours. Thus, the method 40 may be adapted to monitor for long-term trends of the respiratory instability signal.

In an analogous manner to step 12 of method 10, method 40 may be performed iteratively, to thereby iteratively obtain a window of the respiratory instability signal and process the window to determine whether to generate a respiratory instability signal.

The window of a subsequent iteration should begin (i.e. have a start time) after the start time of the window of a previous iteration. In some embodiments, for the sake of simplicity, the window of a subsequent iteration may begin after or at the end time of a window of a previous iteration. This may be required as (long-term) windows may contain a large amount of data, and it may not be reasonably possible to continually store and process overlapping windows. That being said, in some embodiments, the window of a subsequent iteration may begin a predetermined delay time after a window of a previous iteration. To save on processing power, the predetermined delay time may be no less than 5% of the length of the window, for example, no less than 10% or 25% of the length of the window.

In some embodiments, where a window is iteratively obtained, step 42 comprises determining a similarity measure between a current window and a previously obtained window, e.g. a correlation value such as a cross-correlation. Thus, the criterion of step 42 may be that the similarity measure is less than a predetermined similarity value (i.e. that a current window is substantially different to a previous window). This may indicate that the respiratory instability of the subject has significantly changed. Where the similarity measure is a cross correlation, the predetermined similarity value may be no less than 0.65, for example, no less than 0.5, for example, no less than 0.4.

Thus, methods of monitoring the respiratory instability signal general comprise processing the respiratory instability signal according to some criterion to determine whether to generate an alert, e.g. a respiratory instability alert or a respiratory-affecting disease alert. In particular embodiments, a window of the respiratory instability signal is obtained, and processed to determine whether to generate an alert. This allows trends of the respiratory signal to be taken into account, and reduces a likelihood that noise will affect.

Of course, other methods of monitoring the respiratory instability signal to determine whether or not to generate an alert will be apparent to the skilled person. In a simple example, a (respiratory instability) alert may be generated if an instantaneous value of the respiratory instability signal breaches a predetermined threshold. In another example, a (respiratory instability) alert may be generated if a gradient (i.e. derivative) of the respiratory instability signal breaches a certain threshold.

Figure 5 illustrates a use case for the method 40 according to the second embodiment, in which the criterion of step 42 is whether an average magnitude of the window is above a predetermined threshold.

Figure 5 illustrates measures of an average magnitude of a window of the respiratory instability signal in the hours around a time at which clinical suspicion of sepsis is made, being a time at which a clinician first indicated a suspicion of sepsis (at time t=0), e.g. indicated by the ordering of blood samples to identify the presence of pathogens. The data for Figure 5 was taken from cases involving 49 different septic infants, with the error bar indicating the standard error of the mean for said infants. The length of the window is 3 hours, and each window immediately abuts a previous window (so that they do not overlap).

Figure 5 clearly shows how the average magnitude of the window of the respiratory instability signal increases in the hours leading up to clinical suspicion of sepsis, and remain high after that.

Thus, the average magnitude of a window of the respiratory instability signal is a clear indicator or predictor of the onset of sepsis. In other words, the long term trends (as the window is large) of the respiratory instability signal can serve as an indicator of the probability that sepsis will occur in the subject.

In this way, a respiratory-affecting disease alert (e.g. sepsis alert) may be generated if the average of a magnitude of the window of the respiratory instability signal is greater than a predetermined average magnitude threshold.

For example, a predetermined average magnitude threshold set at T_{AV} would generate an alert no fewer than 9 hours before the clinical suspicion of sepsis. This threshold may be increased, e.g. to T_{AV2}, to reduce the occurrence of false positives, at the expense of sensitivity.

Without wishing to be bound by theory, it is believed that diseases such as sepsis dampen the respiratory drive, leading to long term affects in the stability of the respiratory drive, which can be detected through a long-term analysis (e.g. using windows > 1 hour) of the respiratory instability signal. Thus, the onset of sepsis may be detected accurately and in advance of clinical suspicion.

From the foregoing, it is therefore clear that the proposed method of generating and processing a respiratory stability signal enable a prediction of a respiratory failure event (e.g. apnea, sepsis and so on) to be made in advance, and with greater accuracy than existing methods.

Any step of generating an alert described herein may comprise generating an alert signal that triggers or controls a clinician-perceptible output device, such as a display, alarm or vibrating element, that generates any clinician-perceptible output to thereby alert a clinician. Other visual, audio and/or haptic outputs may be used. Thus, generating an alert may comprise generating a clinician-perceptible output to thereby alert the clinician.

Rather than generating an alert, methods may simply present the respiratory instability signal to a clinician (e.g. using a display). This would allow the clinician to easily assess the respiratory instability of the subject intuitively, and with increased accuracy. This could allow, for example, the clinician to make a decision as to a treatment plan (e.g. a level of caffeine to medicate the subject, to overcome apneas) or a discharge-readiness of the subject.

It will be understood that methods may comprise both presenting the respiratory instability alert to the clinician and generating appropriate alerts, e.g. using any above-described embodiment.

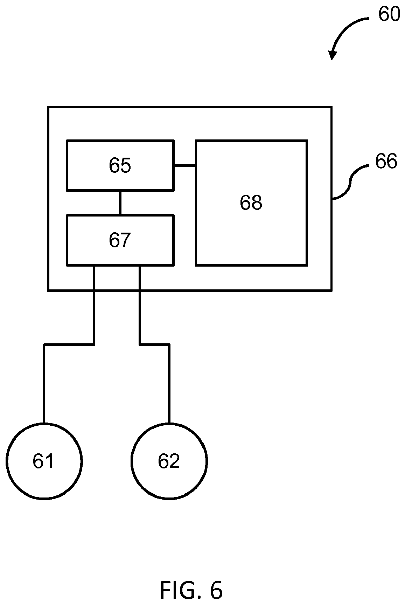

Figure 6 illustrates a subject monitoring system 60 according to an embodiment of the invention.

The subject monitoring system 60 comprises one or more sensors 61, 62 adapted to generate a subject monitoring signal. Examples of suitable sensors include chest impedance sensors and/or pressure sensors.

The subject monitoring system further comprises a processing system 65 for generating a respiratory instability signal representative of a monitored subject's respiratory instability. The processing system 65 may, by itself, form an embodiment of the invention, e.g. to be implemented in a cloud-computing environment.

The processing system 65 is adapted to: receive the subject monitoring signal responsive to respiration of the subject (e.g. from the sensors); and process the subject monitoring signal using a function that derives a measure of periodicity and amplitude regularity during respiration, to thereby generate a respiratory instability signal representative of a monitored subject's respiratory instability.

Thus, the processing system 65 is adapted to perform a previously described method. Indeed, the skilled person would be readily capable of modifying the processing system 65 for carrying out any herein described method. Thus, each step of a flow chart may represent a different action performed by the processing system, and may be performed by a respective module of the processing system.

The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

In some embodiments, as illustrated, the processing system 65 may be integrated into a patient monitor 66. However, hereafter described components of the patient monitor 66 may instead be distributed as separate modules or in different systems.

The patient monitor 66 may also comprise a transceiver 67 adapted to receive signals (e.g. the patient monitoring signal(s)) from the one or more sensors 61, 62. The transceiver 67 may comprise an analogue-to-digital converter for converting the patient monitoring signal(s) into a digital form for processing by the processing system 65.

The patient monitor 66 may also comprise a display 68 or other user interface adapted to display an alert for a clinician. Thus, an alert generated by the processing system 65 (e.g. a respiratory instability alert) may trigger a clinician-perceptible alert (e.g. a red light) being displayed by the display 68. Alternatively or additionally, the patient monitor 66 may comprise other user interfaces, such as speakers or vibrating elements (e.g. mountable on a clinician's wrist) for alerting a clinician in response to an alert generated by the processing system 65.

The display 68 may alternatively or additionally be adapted to present the respiratory instability signal to the clinician. This would allow the clinician to easily assess the respiratory instability of the subject intuitively, and with increased accuracy. This could allow, for example, the clinician to make a decision as to a treatment plan (e.g. a level of caffeine to medicate the subject, to overcome apneas) or a discharge-readiness of the subject.

An assumption has been made throughout this application that an increase in the value of the "respiratory instability signal" indicates increased instability (i.e. complexity or randomness) of respiration. However, embodiments may be reversed so that a decrease in the value of the "respiratory instability signal" indicates increased instability. Reference to "above a ... threshold" for such embodiments should therefore be read as "below a ... threshold", where appropriate, as would be understood by the skilled person.

It will be understood that disclosed methods are preferably computer-implemented methods. As such, there is also proposed the concept of computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A method of generating a respiratory instability signal representative of a monitored subject's respiratory nstability, the method comprising:
receiving a subject monitoring signal responsive to respiration of the subject; and
processing the subject monitoring signal using a function that derives a measure of periodicity and amplitude regularity during respiration, to thereby generate a respiratory instability signal representative of a monitored subject's respiratory instability.

2. The method of claim 1, wherein the function that derives a measure of periodicity and amplitude regularity during respiration is an entropy function.

3. The method of claim 1 or 2, further comprising filtering the subject monitoring signal using a bandpass filter before processing the subject monitoring signal using the function.

4. The method of any of claims 1 to 3, wherein the step of processing the subject monitoring signal comprises iteratively:
obtaining a window of the subject monitoring signal, a window being a windowed portion of the subject monitoring signal captured over a first predetermined length of time; and
processing the window using the function to thereby generate a respiratory instability value for the respiratory instability signal.

5. The method of claim 4, wherein a start time of a window of the subject monitoring signal obtained in any given iteration is after a start time of a window of the subject monitoring signal obtained in an immediately previous iteration.

6. The method of any of claims 4 or 5, wherein the step of obtaining a window of the subject monitoring signal comprises obtaining a most recent portion, of the first predetermined length of time, of the subject monitoring signal.

7. The method of any of claims 4 to 6, wherein the first predetermined length of time is no less than 10 seconds.

8. A method of selectively generating a respiratory instability alert, the method comprising:
generating a respiratory instability signal using the method of any of claims 1 to 7;
monitoring the respiratory instability signal to detect when a parameter of the magnitude of the respiratory instability signal goes above a predetermined threshold;
in response the parameter of the magnitude going above the predetermined threshold:
determining a threshold breach period, being a measure of how long the parameter of the magnitude remains above the predetermined threshold;
generating a respiratory instability alert if the threshold breach period is greater than or equal to a predetermined time period.

9. The method of claim 8, wherein the predetermined time period is no less than 10 seconds, and preferably wherein the parameter of the magnitude is a value of the magnitude of the respiratory instability signal.

10. A method of selectively generating a respiratory-affecting disease alert, the method comprising:
generating a respiratory instability signal using the method of any of claims 1 to 7;
obtaining a window of the respiratory instability signal, the window of the respiratory instability signal being a windowed portion of the respiratory instability signal over a second predetermined length of time;
determining whether the window of the respiratory instability signal meets a predetermined criterion; and
generating a respiratory-affecting disease alert if the window of the respiratory instability signal meets the predetermined criterion.

11. The method of claim 10, wherein the step of determining whether the window of the respiratory signal meets the predetermined criterion comprises:
taking an average of the magnitude of the window of the respiratory instability signal; and
determining that the window of the respiratory signal meets the predetermined criterion if the average of the magnitude of the respiratory instability signal is greater than a predetermined average magnitude threshold.

12. The method of claim 10 or 11, wherein the second predetermined length of time is no less than 1 hour.

13. A computer program comprising code means for implementing the method of any one of claims 1 to 12 when said program is run on a computer.

14. A processing system for generating a respiratory instability signal representative of a monitored subject's respiratory instability, the processing system being adapted to:
receive a subject monitoring signal responsive to respiration of the subject; and
process the subject monitoring signal using a function that derives a measure of periodicity and amplitude regularity during respiration, to thereby generate a respiratory instability signal representative of a monitored subject's respiratory instability.

15. A subject monitoring system for generating a respiratory instability signal, the patient monitoring system comprising:
one or more subject monitoring sensors adapted to generate a subject monitoring signal responsive to respiration of the subject; and
the processing system of claim 14 adapted to receive the subject monitoring system generating by the one or more subject monitoring sensors.
